# EUROPEAN PATENT SPECIFICATION

(11) **EP 1 078 023 B1**
(45) Date of publication and mention of the grant of the patent: **05.01.2011**
(21) Application number: 99915319.0
(22) Date of filing: 07.04.1999
(51) Int. Cl.: C09K 11/06, G01N 21/64, C09B 23/00

(54) **LUMINESCENT COMPOUNDS**
LUMINESZIERENDE VERBINDUNGEN
COMPOSES LUMINESCENTS

(30) Priority: 08.04.1998 DE 19815659; 01.05.1998 US 83820 P
(43) Date of publication of application: 28.02.2001
(60) Divisional application: 07102880.7
(73) Proprietor: Luminex Corporation, Austin TX 78727 (US)
(72) Inventor: Luminex Corporation, Austin TX 78727 (US)
(74) Representative: Harrison Goddard Foote
(86) International application number: PCT/US1999/007627
(87) International publication number: WO 1999/051702

(56) References cited:
- WO-A-96/41144
- US-A- 3 996 345
- US-A- 3 998 943
- US-A- 4 883 867
- US-A- 5 101 015
- E. TERPETSCHNIG ET AL.: "Synthesis, spectral properties and photostabilities of symmetrical and unsymmetrical squaraines; a new class of fluorophores with long-wavelength excitation and emission" ANALYTICA CHIMICA ACTA, vol. 282, 1993, pages 633-641,

## Description

### Field of the Invention

The invention relates to luminescent compounds based on squaric acid.

### Background of the Invention

A luminescent compound, or luminophore, is a compound that emits light. A luminescence method, in turn, is a method that involves detecting light emitted by a luminophore, and using properties of that light to understand properties of the luminophore and its environment. Luminescence methods may be based on chemiluminescence and/or photoluminescence, among others, and may be used in spectroscopy, microscopy, immunoassays, and hybridization assays, among others.

Photoluminescence is a particular type of luminescence that involves the absorption and subsequent re-emission of light. In photoluminescence, a luminophore is excited from a low-energy ground state into a higher-energy excited state by the absorption of a photon of light. The energy associated with this transition is subsequently lost through one or more of several mechanisms, including production of a photon through fluorescence or phosphorescence.

Photoluminescence may be characterized by a number of parameters, including extinction coefficient, excitation and emission spectrum, Stokes' shift, luminescence lifetime, and quantum yield. An extinction coefficient is a wavelength-dependent measure of the absorbing power of a luminophore. An excitation spectrum is the dependence of emission intensity upon the excitation wavelength, measured at a single constant emission wavelength. An emission spectrum is the wavelength distribution of the emission, measured after excitation with a single constant excitation wavelength. A Stokes' shift is the difference in wavelengths between the maximum of the emission spectrum and the maximum of the absorption spectrum. A luminescence lifetime is the average time that a luminophore spends in the excited state prior to returning to the ground state. A quantum yield is the ratio of the number of photons emitted to the number of photons absorbed by a luminophore.

Luminescence methods may be influenced by extinction coefficient, excitation and emission spectra, Stokes' shift, and quantum yield, among others, and may involve characterizing fluorescence intensity, fluorescence polarization (FP), fluorescence resonance energy transfer (FRET), fluorescence lifetime (FLT), total internal reflection fluorescence (TIRF), fluorescence correlation spectroscopy (FCS), fluorescence recovery after photobleaching (FRAP), and their phosphorescence analogs, among others.

Luminescence methods have several significant potential strengths. First, luminescence methods may be very sensitive, because modem detectors, such as photomultiplier tubes (PMTs) and charge-coupled devices (CCDs), can detect very low levels of light. Second, luminescence methods may be very selective, because the luminescence signal may come almost exclusively from the luminophore.

Despite these potential strengths, luminescence methods suffer from a number of shortcomings, at least some of which relate to the luminophore. For example, the luminophore may have an extinction coefficient and/or quantum yield that is too low to permit detection of an adequate amount of light. The luminophore also may have a Stokes' shift that is too small to permit detection of emission light without significant detection of excitation light. The luminophore also may have an excitation spectrum that does not permit it to be excited by wavelength-limited light sources, such as common lasers and arc lamps. The luminophore also may be unstable, so that it is readily bleached and rendered nonluminescent. The luminophore also may have an excitation and/or emission spectrum that overlaps with the well-known autoluminescence of biological and other samples; such autoluminescence is particularly significant at wavelengths below about 600 nm. The luminophore also may be expensive, especially if it is difficult to manufacture.

Terpetschnig et al., Anal. Biochem. 217, 197-204 (1994) discloses the synthesis and fluorescence characterisation of a squaraine-N-hydroxysuccinimide ester and a sulfobutylsquaraine-N-hydroxysuccinimide ester.

WO 96/41144 describes marker components that comprise a fluorophore moiety coupled to one or more polyoxyhydrocarbyl moieties. Squarylium dyes are mentioned as possible fluorophores and one squarylium compound is exemplified in Fig. 7v.

The reader of this patent is referred to the following publications: JOSEPH R. LAKOWICZ, PRINCIPLES OF FLUORESCENCE SPECTROSCOPY (1983); RICHARD J. LEWIS, SR., HAWLEY'S CONDENSED CHEMICAL DICTIONARY (12th ed. 1993).

### Summary of the Invention

The invention provides photoluminescent compounds and methods of synthesizing and using photoluminescent compounds, among others.

The compounds relate generally to the following structure: 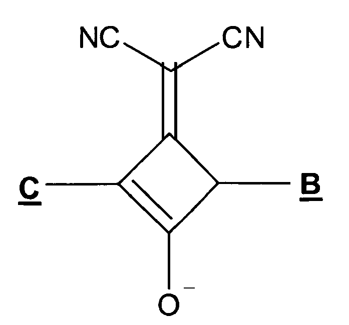 wherein:
(a) each of B and C is selected from the group consisting of W¹ and W², wherein W¹ and W² are given by 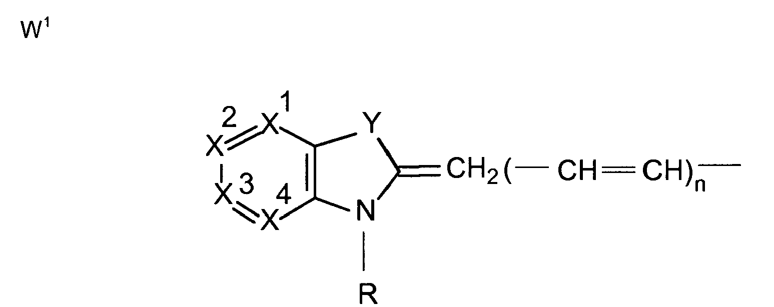 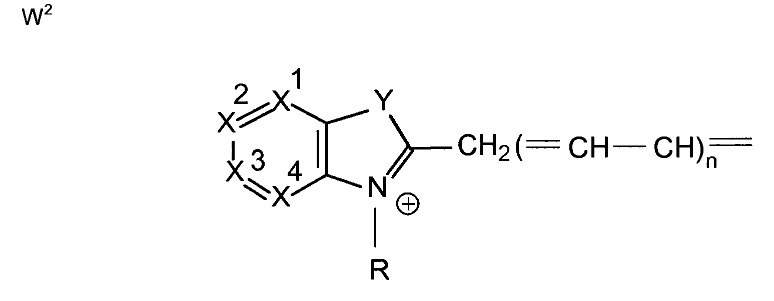
(b) one of B and C is W¹ and the other of B and C is W²;
(c) n is independently selected for each of B and C from the group consisting of 0, 1, and 2;
(d) Y is independently selected for each of B and C from the group consisting of O, S, Se, Te, N-R^{h}, and C(Rⁱ)(R^{j}), wherein R^{h} is selected from the group consisting of H, aliphatic groups, alicyclic groups and aromatic groups and wherein each of Rⁱ and R^{j} is selected from the group aliphatic groups;
(e) R is independently selected for each of B and C from the group consisting of H, aliphatic groups, alicyclic groups and aromatic groups; and
(f) each of X¹, X², X³, and X⁴ is independently selected for each of B and C from the group consisting of N, O, S, and C-R^{k}, wherein R^{k} is selected from the group consisting of H, F, Cl, Br, I, aliphatic groups, alicyclic groups, aromatic groups and parts of a substituted condensed aromatic or heterocyclic ring;
wherein the photoluminescent compound is covalently or non-covalently associated with a carrier that is a bead.

In some embodiments, the compound includes at least one S. In other embodiments, the compound includes at least one heteroatom in X¹ through X⁴ of W¹ or W². In yet other embodiments, the compound includes a carrier. The methods relate generally to the use of photoluminescent compounds, as described above.

The nature of the invention will be understood more readily after consideration of the drawing, chemical structures, and detailed description of the invention that follow.

### Brief Description of the Drawing

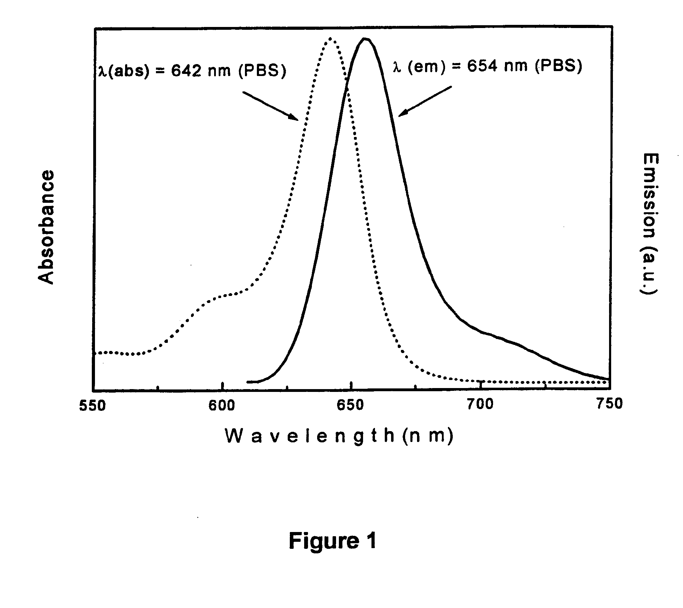

Figure 1 is a graph showing excitation (dotted line) and emission (solid line) spectra for (13)-HSA in phosphate-buffered saline (PBS).

### Abbreviations

The following abbreviations, among others, may be used in this application:

| | |
|---|---|
| BSA | bovine serum albumin |
| Bu | butyl |
| DCC | dicyclohexylcarbodiimide |
| DMF | dimethylformamide |
| D/P | dye-to-protein ratio |
| Et | ethyl |
| g | grams |
| h | hours |
| HSA | human serum albumin |
| hCG | human chorionic gonadotropin |
| L | liters |
| m | milli (10⁻³) |
| M | molar |
| Me | methyl |
| mol | moles |
| M.P. | melting point |
| n | nano (10⁻⁹) |
| NHS | N-hydroxysuccinimide |
| NIR | near infrared region |
| PBS | phosphate-buffer saline |
| Prop | propyl |
| TMS | tetramethylsilane |
| TSTU | N, N, N', N'-tetramethyl(succinimido)uronium tetrafluoroborate |
| µ | micro (10⁻⁶) |

### Detailed Description of the Invention

The invention relates generally to photoluminescent compounds and to methods of using such compounds. These photoluminescent compounds may be useful in both free and conjugated forms, as probes, labels, and/or indicators. This usefulness may reflect in part enhancement of one or more of the following: quantum yield, Stokes' shift, extinction coefficients, and photostability. This usefulness also may reflect excitation and emission spectra in relatively inaccessible regions of the spectrum, including the red and near infrared.

The remaining discussion includes (1) an overview of structures, (2) an overview of synthetic methods, and (3) a series of illustrative examples.

### Overview of structures

The photoluminescent compounds and their synthetic precursors generally comprise the following structure:

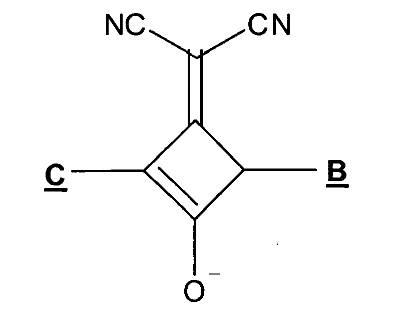

The various symbols have the previously stated meanings.

Photoluminescent compounds. In the photoluminescent compounds, B and C are chosen from W¹ and W², one of B and C is W¹, and one of B and C is W².

Other features of the compounds are as previously described.

Carrier groups. The photoluminescent compounds of the invention are covalently or noncovalently associated with a carrier that is a bead. Covalent association may occur through various mechanisms, including a reactive group, and may involve a spacer for separating the photoluminescent compound or precursor from the carrier. Noncovalent association also may occur through various mechanisms, including incorporation of the photoluminescent compound or precursor into or onto a matrix, such as a bead, or by nonspecific interactions, such as hydrogen bonding, ionic bonding, or hydrophobic interactions.

Alicyclic groups include groups of organic compounds characterized by arrangement of the carbon atoms in closed ring structures sometimes resembling boats, chairs, or even bird cages. These compounds have properties resembling those of aliphatics and should not be confused with aromatic compounds having the hexagonal benzene ring. Alicyclics comprise three subgroups: (1) cycloparaffins (saturated), (2) cycloolefins (unsaturated with two or more double bonds), and (3) cycloacetylenes (cyclynes) with a triple bond. The best-known cycloparaffins (sometimes called naphthenes) are cyclopropane, cyclohexane, and cyclopentane; typical of the cycloolefins are cyclopentadiene and cyclooctatetraene. Most alicyclics are derived from petroleum or coal tar, and many can be synthesized by various methods.

Aliphatic groups include groups of organic compounds characterized by straight- or branched-chain arrangement of the constituent carbon atoms. Aliphatic hydrocarbons comprise three subgroups: (1) paraffins (alkanes), which are saturated and comparatively unreactive; (2) olefins (alkenes or alkadienes), which are unsaturated and quite reactive; and (3) acetylenes (alkynes), which contain a triple bond and are highly reactive. In complex structures, the chains may be branched or cross-linked.

Aromatic groups include groups of unsaturated cyclic hydrocarbons containing one or more rings. A typical aromatic group is benzene, which has a 6-carbon ring containing three double bonds. Most aromatics are highly reactive and chemically versatile. Most aromatics are derived from petroleum and coal tar. Some 5-membered cyclic compounds, such as the furan group (heterocyclic), are analogous to aromatic compounds.

Heterocyclic rings include closed-ring structures, usually of either 5 or 6 members, in which one or more of the atoms in the ring is an element other than carbon, e.g., sulfur, nitrogen, etc. Examples include pyridine, pyrole, furan, thiophene, and purine.

### Overview of synthesis and characterization

The synthesis of photoluminescent compounds according to the invention typically is achieved in a multi-step reaction, starting with the synthesis of a methylene base. The synthesis of suitable bases may proceed based on literature or novel methods. Generally, the spectral properties of the photoluminescent compounds, including excitation and emission wavelength, are strongly dependent on the type of methylene base used. Typical starting materials include benzindoles, benzoselenzoles, benzoxazoles, benzimidazoles, etc., and squaric acid. Squaric acid is a dibasic acid that undergoes a series of nucleophilic substitution reactions with various reagents, including amines, phenols, and CH-acidic compounds such as 1,2,3,3-tetramethl-benzindole. The squaraine bridge in the resulting compounds stabilizes the conjugated chain and shifts the excitation and emission wavelength of these dyes to the red as compared to cyanine-based dyes. In particular, the exchange of the oxygen in the squaraine moiety by a methylen (=CR₂)-functionality is shown here to be a pathway to a new group squaraine dyes with useful fluorescence properties.

In the examples that follow this section, the synthesis and spectral characterization of several long-wavelength fluorescent labels based on squaraine dyes is presented, including some reactive versions. These dyes may include a cyanine-type chromophore and a squarate bridge. To achieve water-solubility, sulfonic acid or other groups may be introduced into the heterocyclic ring systems, and to permit covalent attachment to proteins, reactive N-hydroxysuccinimide ester (NHS ester) or other forms may be synthesized. To modify the spectral properties of the dyes, dicyanomethylen-substituted versions of the squaraines were synthesized and tested for their potential use for labeling of biopolymers. The squaraine-based markers exhibit lower quantum yields in water (φ = 0.15) and very high quantum yields (φ = 0.5 - 0.7) when bound to biomolecules. The absorption and emission wavelengths can be tuned by substitution of the squaraine ring or by introducing heteroatoms into the heterocyclic moiety. Thus, the indolenine-squaraines absorb around 635 to 640 in water and at approximately 645 to 650 nm when bound to proteins. The absorption and emission spectra of benzothiazolium and benzoselenzolium squaraines on the other hand are shifted towards longer wavelengths. Typical emission wavelengths for such squaraine dyes are around 680 nm to 690 nm for benzothiazole dyes and beyond 700 nm for benzoselenzole derivatives. Importantly, the Stokes' shift increases in these longer wavelength-emitting dyes, which ultimately increases the sensitivity of a fluorescent measurement.

The resulting dyes show absorption and emission maxima beyond 600 nm and their wavelength can be tuned by changing the heterocyclic moiety and/or the substitution on the squaraine ring system. The Stokes' shift of the methylene derivatives of symmetric squaraines (15) is increased more than 2.5 times relative to the Stokes' shift of the analogous oxygen-containing squaraines (8) and (3b). In addition, the replacement of C=O by C=C in example **(15)** results in a bathochromic shift of both, the absorption and the emission properties of these dyes. A further increase of the Stokes' shift can be achieved by introducing asymmetry into the molecule. Thus, the asymmetric versions **(15)** are expected to have even higher Stokes' shifts.

The substitution of one oxygen of the central squarate bridge with CH-acidic reagents e.g. dicyanomethane, HOOC-(CH₂)-COOH, or ROOC-(CH₂)-CN leads to the group of luminescent methylenesquaraine derivatives. As compared to the basic squaraines these compounds have red-shifted excitation and emission properties and larger Stokes' shifts. The absorption and emission maxima of a representative reactive dye **(15)** (example 7) were found to be 667 nm and 685 nm in PBS, respectively.

Example 8 describes synthetic pathways to unsymmetrical thiosquaraine and methylenesquaraine dyes. The thiosquaraine compounds of this example do not form part of the invention as claimed. The key intermediates for this subgroup of squaraine dyes are mono-substituted thiosquaraine and methylenesquaraine derivatives, which are synthesized by reacting the 1,3-dithiosquaric acid disodium salt **(2c)** or 4-dicyanomethylene-2,3-dibutyl squarate **(2d)** with 1 equivalent of indolenine **(1a).** Subsequently the intermediate is reacted with one equivalent of a different methylene base. The synthesis of unsymmetrical squaraine dyes allows access to mono-functional reactive squaraine dyes (Scheme 1). Such dyes show improved labelling performance because of reduced crosslinking with proteins.

Example 9 shows structures of thiosquaraine and methylenesquaraine based dyes which should further demonstrate the variety of structures which can be synthesized from this invention. Only the dicyanomethylene-squaraine compounds of this example form part of the invention as claimed. Most of the representative structures are based on reactive water soluble dyes showing different substitution patterns on the central bridge. Two of the structures contain phosphoramidite linkages for direct coupling used in solid support DNA or RNA synthesis. Sulfonated and non-sulfonated versions of squaraine dyes can be used for the synthesis of phosphoramidites. The phosphoramidite linkage can either be incorporated in the heterocyclic bases or can be attached to the central squarate ring via activation of a carboxyl-cyano methylene function to an NHS ester and reacting it with an amino-alcohol spacer group. The introduced hydroxyl function is then converted by standard procedures into a phosphoramidite.

### EXAMPLE 1

### Synthesis of precursors

This section describes the synthesis of various precursors. p-hydrazinobenzenesulfonic acid (Illy et al., J. Org. Chem. 33, 4283-4285 (1968)), 1-(ε-carboxypentyl)-2,3,3-trimethylindolenium-5-sulfonic acid potassium salt **(1a),** 2,3,3-trimethylindole-5-sulfonic acid potassium salt (Mujumdar et al., Bioconj. Chem. 4, 105-111 (1993)) **(1b),** and 1,2,3,3-tetramethylindoleninium-5-sulfonate **(1c)** were synthesized using literature procedures. 1,3-dithiosquaric acid disodium salt **(2c)** and dicyanomethylenedimethylsquarate **(2d)** were synthesized according to G. Seitz et al., Chem. Ber. 112, 990-999 (1979), and B. Gerecht et al., Chem. Ber. 117, 2714-2729 (1984), respectively.

### Preparation of 1-(ε-carboxypentyl)-2,3,3-trimethylindolenium-5-sulfonic acid potassium salt (1a)

### p-Hydrazinobenzenesulfonic acid

33 g of sodium carbonate was added to a suspension of 104 g (0.6 mol) of p-aminobenzenesulfonic acid in 400 mL of hot water. The solution was cooled to 5°C in an ice-bath, and 70 g of concentrated sulfuric acid were added slowly under rapid stirring. A solution of 42 g of sodium nitrite in 100 mL of water was then added under cooling. A light yellow diazo-compound precipitate formed, which was filtered and washed with water, but not dried.

The wet diazo-compound was added under stirring and cooling (5°C) to a solution of 170 g of sodium sulfite in 500 mL of water. The solution, which turned orange, was stirred under cooling for 1 h, and then heated to reflux. Finally, 400 mL of concentrated hydrochloric acid were added. The solution turned yellow, and the product precipitated as a white solid. For complete decoloration, 1-2 g of powdered zinc were added. The reaction mixture was cooled overnight, and the precipitate was filtered, washed with water, and dried in an oven at 100°C.

Yield: 96 g (85%), white powder; M.P. = 286°C (Lit. = 285°C); R_{f}: 0.95 (RP-18, water:MeOH 2:1).

### Preparation of 2,3,3-trimethylindole-5-sulfonic acid, potassium salt (1b)

18.2 g (0.12 mol) of p-hydrazinobenzenesulfonic acid and 14.8 g (0.17 mol) of isopropylmethylketone were stirred in 100 mL of glacial acetic acid at room temperature for 1 h. The mixture was then refluxed for 4 h. The mixture was cooled to room temperature, and the resulting pink solid precipitate was filtered and washed with ether.

The precipitate was dissolved in methanol, and a concentrated solution of potassium hydroxide in 2-propanol was added until a yellow solid completely precipitated. The precipitate was filtered, washed with ether, and dried in a desiccator over P₂O₅.

Yield: 20.4 g (71%), off-white powder; M.P. = 275°C; R_{f}: 0.40 (silica gel, isopropanol:water:ammonia 9:0.5:1).

### 1-(ε-carboxypentyl)-2,3,3-trimethylindolenium-5-sulfonic acid, potassium salt (1a)

15.9 g (57 mmol) of 2,3,3-trimethylindolenium-5-sulfonic acid potassium salt and 12.9 g (66 mmol) of 6-bromohexanoic acid were refluxed in 100 mL of 1,2-dichlorobenzene for 12 h under a nitrogen atmosphere. The solution was cooled to room temperature, and the resulting pink precipitate was filtered, washed with chloroform, and dried.

Yield: 15.8 g (58%), pink powder; R_{f}: 0.75 (RP-18, MeOH:water 2:1).

### Synthesis of 1,2,3,3-tetramethylindoleninium-5-sulfonate (1c)

1.1 g of 2,3,3-trimethylindoleninium-5-sulfonate were suspended in 30 mL of methyl iodide. The reaction mixture was heated to boiling for 25 h in a sealed tube. After the mixture was cooled, excess methyl iodide was decanted, and the residue was suspended in 50 mL of acetone. The solution was filtered, and the residue was dried in a desiccator over CaCl₂. The resulting light yellow powder was used without further purification.

Yield: 90%, light yellow powder.

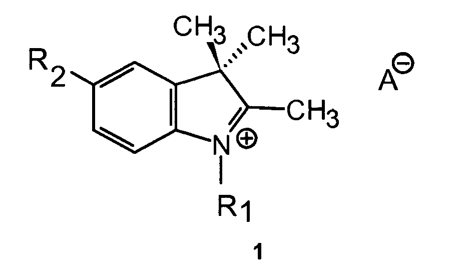

| **1** | R₁ | R₂ | A⁻ |
|---|---|---|---|
| a | (CH₂)₅COOH | SO₃K | I |
| b | - | - | - |
| c | CH₃ | SO₃K | I |

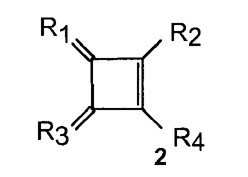

| **2** | R₁ | R₂ | R₃ | R₄ |
|---|---|---|---|---|
| a | O | O | OH | OH |
| b | O | O | OC₄H₉ | OC₄H₉ |
| c | S | O | S- Na⁺ | O- Na⁺ |
| d | O | CH₂(CN)₂ | OCH₃ | OCH₃ |

### EXAMPLE 2

### Synthesis of 2,4-Bis[N-(butoxycarbonylpentyl)-3,3-dimethyl-5-sulfo-2-indolinyl idene methyl cyclobutenediylium-3-dicyanomethylene-1-oxolate (15)

### 3-Dicyanomethylene-2,4-dibutyl-squarate (2d)

3-Dicyanomethylene-2,4-dibutyl-squarate **(2d)** was prepared according to Gerecht et al. (1984).

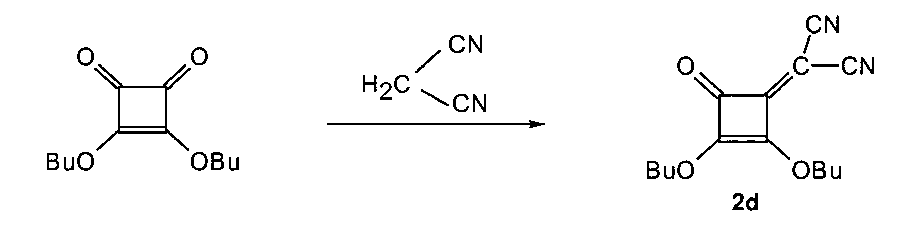

2.16 mL (10 mmol) of squaric acid dibutylester **(2c)** were dissolved in 40 mL of THF. 660 mg (10 mmol) of malonedinitrile were added under stirring. A solution of 1.64 mL of triethylamine in 3 mL of THF was then added, and the mixture was stirred at room temperature for 15 h. The solvent was removed under reduced pressure, and a yellow-brown oil remained. The raw product is purified by MPLC using silica gel as the stationary phase and methanol as eluent.

Yield: 173 mg (63%) of **(2d).**

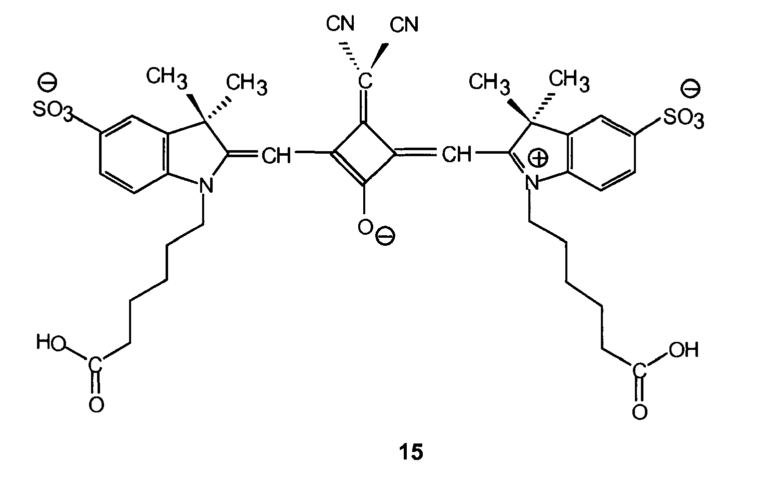

472 mg of 1-(ε-carboxypentyl)-2,3,3-trimethylindolenium-5-sulfonic acid potassium salt **(1a)** and 137 mg of malondinitrile squaric acid dibutylester **(2d)** were refluxed in 25 mL of butanol:toluene (1:1, v:v) for 4 h using a Dean-Stark trap. After the mixture was cooled to room temperature, the solvents were removed in vacuum, and the raw product was triturated with ether and dried. The raw product was purified by preparative thin-layer chromatography on RP-18 glass plates using a methanol/water mixture (2/1, v:v) as eluent. The blue-green band with an R_{f} of 0.55 was collected.

Yield: 32%; FAB-MS m/e calculated for C₄₁H₄₄N₄O₁₁S₂K₂ (M²⁻ ) 832.9, found 633.2. IR (KBr): 2100 cm⁻¹ (CN). ¹H-NMR (D₂O): δ 8.00 (2H, s), 7.90 (2H, d), 7.75 (2H, d), -CH= is exchanged, 4.45 (4H, t), 2.10(4H, t), 1.85 (4H, m), 1.55 (4H, m), 1.45 (12H, s), 1.35 (4H, m); λₘₐₓ(abs) = 667 nm (PBS), λₘₐₓ(em) = 685 nm (PBS), (4%); λₘₐₓ(abs) = 687 nm (PBS + HSA), λₘₐₓ(em) = 704 nm (PBS + HSA), (8%), ε = 110.000 L/mol*cm (H₂O).

### EXAMPLE 3

The thiosquaraine compounds of this example do not form part of the invention as claimed, but are retained as useful background information.

### Asymmetric thiosquaraine and dicyanomethylene-squaraines

### Synthesis of mono-substituted thiosquaraine and dicyanomethylen-squaraine derivatives

The following are important intermediates for the synthesis of asymmetric squaraine dyes and can be synthesized using either squaric acid or squaric ester derivatives as starting materials. A general procedure for the synthesis of two classes of asymmetric squaraine analogs is given below.

1 mmol of 1-(ε-carboxypentanyl)-2,3,3-trimethylindolenium-5-sulfonate was dissolved in 10 mL of ethanol containing 100 µL of triethylamine. The mixture was warmed to 40°C, and 1.1 mmol of either dicyanomethylene-squaric acid dibutyl ester **(2d)** or 1,3-dithio squaric acid disodium salt **(2c)** were added in small portions. The reaction mixture was then heated to 60°C and stirred for 4 h. After the mixture was cooled to room temperature, the solvent was removed under reduced pressure. The residue was redissolved in ethanol, 5 mL of 0.1 M HCl were added and the mixture was refluxed for 10 min. After cooling, the solvents were removed, and the residue was washed several times with either ether or chloroform.

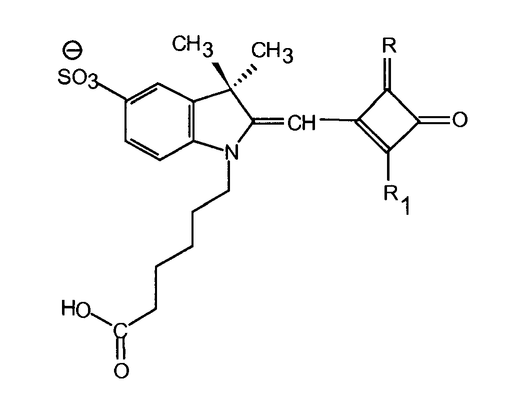

| | | |
|---|---|---|
| Squaraine-derivative | R | R1 |
| Dicyano-methylene | C(CN)₂ | OH |
| Dithio- | S | SH |

### General synthesis procedure for asymmetric thiosquariane and methylenesquariane dyes

The above intermediates were heated under reflux with 1 mmol of 1,2,3,3-tetramethylindoleninium-5-sulfonate in a butanol:toluene mixture (1:1 v/v) for 5 h using a Dean-Stark trap. After cooling, the solvents were removed under reduced pressure. The product can be purified on preparative TLC (RP-18 F₂₅4s) using methanol:water (2:1 v:v) as the eluent.

Yield: 25 - 30% of asymmetric compound.

### Synthesis scheme for an asymmetric thiosquaraine dye

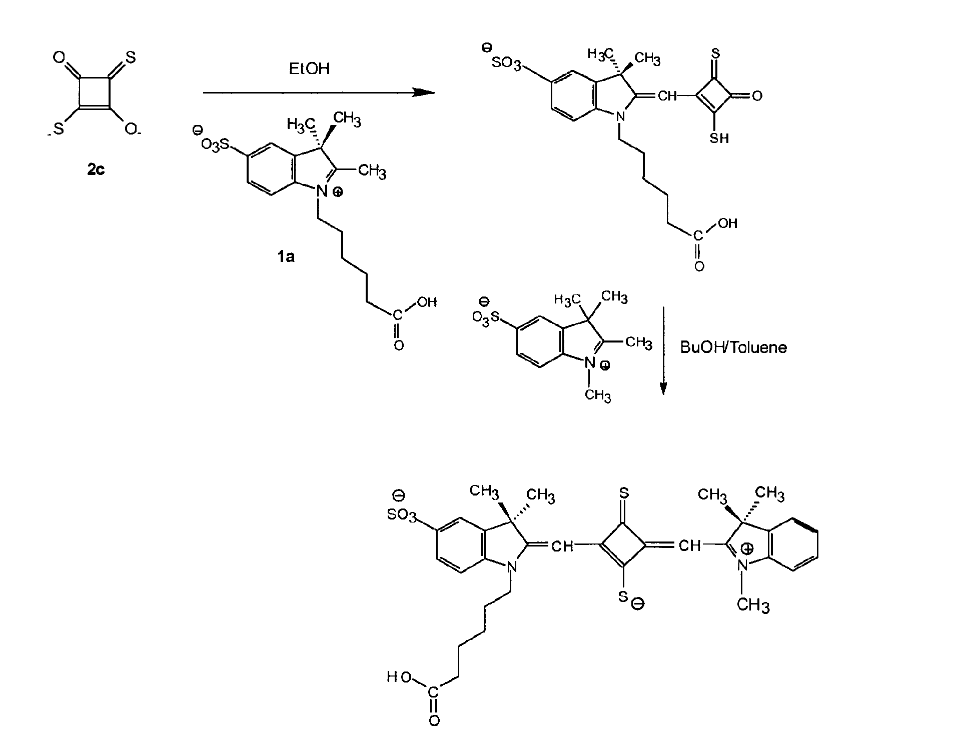

### EXAMPLE 4

Only the dicyanomethylene - squaraine compounds of this example form part of the invention as claimed. The remaining compounds are retained as useful background information.

### Additional structures of thiosquariane and methylenesquaraine dyes:

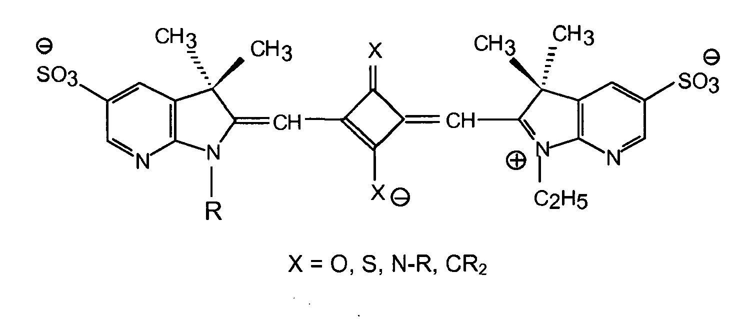

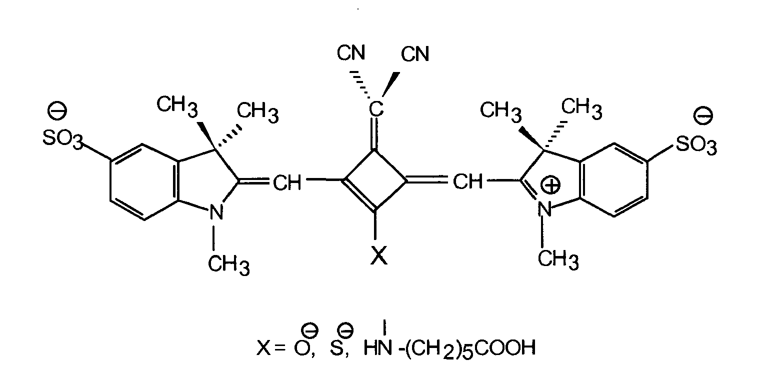

The final example shows a central squaraine ring (X= O-, S-) converted so that it contains a reactive functional group for covalent attachment to carrier molecules. To introduce an amino-function into a squaraine ring (X = O-, S-), the squaraine or substituted squaraine is converted into a mono-ester version (X = OMe, OBu), where Me is methyl and Bu is butyl. The mono-ester is then reacted with the amine or an amino acid derivative to give the amino derivative. This mechanism offers a convenient way of converting a nonreactive squaraine dye into a reactive analog.

### EXAMPLE 5

### General Protein Labeling Procedures and Determination of Dye-to-Protein Ratios

Protein labeling reactions were carried out using a 50 mM bicarbonate buffer (pH 9.1). A stock solution of 1 mg of dye in 100 µL of anhydrous DMF was prepared. 10 mg of protein were dissolved in 1 mL of 100 mM bicarbonate buffer (pH 9.1). Dye from the stock solution was added, and the mixture was stirred for 24 h at room temperature.

Unconjugated dye was separated from labeled proteins using gel permeation chromatography with Sephadex G50 (0.5 cm x 20 cm column) and a 22 mM phosphate buffer solution (pH 7.3) as the eluent. The first colored band contained the dye-protein conjugate. A later blue band with a much higher retention time contained the separated free dye. A series of labeling reactions as described above were set up to obtain different dye-to-protein ratios. Compared to the free forms, the protein-bound forms of the dyes show distinct changes in their spectral properties.

Protein concentration was determined using the BCA Protein Assay Reagent Kit from Pierce (Rockford, IL). The dye-to-protein ratio (D/P) gives the number of dye molecules covalently bound to the protein.

### Covalent attachment of NHS-ester (14) to polyclonal anti-HSA

385 µL (5.2 mg/mL) of anti-HSA were dissolved in a 750 µL bicarbonate buffer (0.1 M, pH 9.0). 1 mg of NHS-ester **(14)** was dissolved in 50 µL of DMF and slowly added to the above-prepared protein solution with stirring. After 20 h of stirring, the protein-conjugate was separated from the free dye using Sephadex G50 and a phosphate buffer (22 mM, pH 7.2). The first blue band that was isolated contained the labeled conjugate.

### Conjugation of (14) to HSA

0.5 mg of **(14)** in 50 µL of DMF were slowly added to a stirred solution of 5 mg of HSA in 750 µL of bicarbonate buffer (0.1 M, pH 9.0). The mixture was stirred for another 6 h at room temperature. The mixture was dialyzed against a phosphate buffer (22 mM, pH 7.2) using a dialysis membrane (1500 FT, Union Carbid) with a cutoff of 10.000.

Analysis: λₘₐₓ(abs) = 642 nm (PBS); λmax(em) = 654 nm (PBS).

Similar reactions were performed using the other reactive dyes.

### Fluorescence decay times of various dyes and their conjugates:

The following table shows fluorescence decay times of various dyes and their conjugates. The experimental conditions included (1) excitation at 600 nm, using a rhodamine B dye laser, (2) emission observed at 660 nm, using an interference filter having a 10 nm band pass, and (3) temperature of 20°C.

| **Sample** | **Decay time [ns]** | **Amplitude** | **Fractional Intensity** | **Mean lifetime [ns]** | **Chi square** |
|---|---|---|---|---|---|
| **3a** | 0.21 | 0.752 | 0.496 | 0.43 | 3.7 |
| | 0.65 | 0.248 | 0.504 | | |
| **4** | 0.20 | 0.558 | 0.286 | 0.51 | 3.8 |
| | 0.64 | 0.442 | 0.714 | | |
| **3b-HSA** | 0.18 | 0.676 | 0.142 | | |
| | 0.96 | 0.089 | 0.097 | 2.26 | 1.7 |
| | 3.81 | 0.235 | 0.761 | | |
| **13-HSA** | 0.011 | 0.865 | 0.036 | | |
| | 0.768 | 0.068 | 0.201 | 2.44 | 5.22 |
| | 2.99 | 0.067 | 0.764 | | |
| | | | | | |
| | | | | | |
| **Cy5** | 1.02 | 1 | 1 | 1.01 | 2.1 |
| **Cy5-hCG** | 0.16 | 0.408 | 0.071 | 1.33 | 2.8 |
| | 1.41 | 0.592 | 0.929 | | |

### Spectral properties and dye-to-protein ratios for various reactive squaraine dyes and their conjugates:

Spectral properties and dye-to-protein ratios were determined for various reactive squaraine dyes and their conjugates. Figure 1 shows absorption (excitation) and emission spectra for (**13**)-HSA in PBS. The following table summarizes data for (**13**)-HSA and various other reactive squaraine dyes and their conjugates in PBS.

| Squaraine | λₘₐₓ(abs) [nm] | λₘₐₓ (em) [nm] | ε [L(mol*cm)] | Q.Y. [%] | D/P [mol/mol] |
|---|---|---|---|---|---|
| **3b** | 635 | 642 | 180.000 | 13 | --- |
| **3b-HSA** | 642 | 653 | --- | 60 - 70 | 1 |
| **6a** | 627 | 647 | 100.000 | 3 | --- |
| **7** | 634 | 646 | 120.000 | 13 | |
| **7-HSA** | 635 | 660 | --- | 50 | 0.5 |
| **13** | 630 | 649 | 66.000 | 5 | --- |
| **13-HSA** | 642 | 654 | --- | 60 - 70 | 0.8 |
| **15** | 667 | 685 | 110.000 | 4 | --- |
| **15-HSA** | 685 | 704 | --- | n.d. | n.d. |

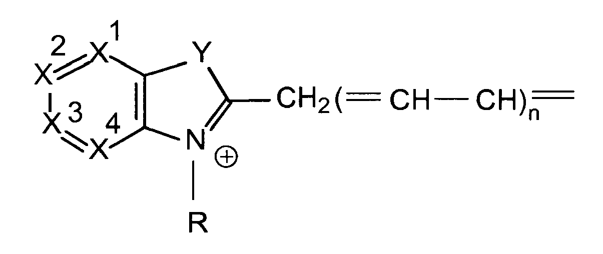

### Description of applications of the invention

In addition to the compounds the invention provides also a number of methods for utilizing these compounds in various assay formats.

The assay may be a competitive assay that includes a recognition moiety, a binding partner, and an analyte. Binding partners and analytes may be selected from the group consisting of biomolecules, drugs, and polymers. In some competitive assay formats, one or more components are labeled with photoluminescent compounds in accordance with the invention. For example, the binding partner may be labeled with such a photoluminescent compound, and the displacement of the compound from an immobilized recognition moiety may be detected by the appearance of fluorescence in a liquid phase of the assay. In other competitive assay formats, an immobilized enzyme may be used to form a complex with the fluorophore-conjugated substrate.

The binding of antagonists to a receptor can be assayed by a competitive binding method in so called ligand/receptor assays. In such assays, a labeled antagonist competes with an unlabelled ligand for the receptor binding site. One of the binding partner can be, but not necessarily does has to be immobilized. Such assays can also be performed in microplates. Immobilization can be achieved via covalent attachment to the well wall or to the surface of beads.

Other preferred assay formats are immunological assays. There are several types assay formats. Competitive binding assays, in which labeled and unlabeled antigens compete for the binding sites on the surface of an antibody (binding material). Typically, there are incubation times required to provide sufficient time for equilibration. Such assays can be performed in a heterogeneous or homogeneous fashion.

Sandwich assays use secondary antibodies and access binding material is removed from the analyte by a washing step.

Other types of reactions include binding between avidin and biotin, protein A and immunoglobulins, lectins and sugars (e.g., concanavalin A and glucose).

Photoluminescent compounds described here also may be used to sequence nucleic acids and peptides. For example, fluorescently labeled oligonucleotides may be used to trace DNA fragments.

Other applications of labeled DNA primers besides for DNA sequencing are in fluorescence *in-situ* hybridization methods (FISH) and for single nucleotide polymorphisms (SNPs) applications.

Multicolor labeling experiments allow different biochemical parameters to be monitored simultaneously. For this purpose, two or more fluorophores are introduced into the biological system to report on different biochemical functions. The technique can be applied to fluorescence *in-situ* hybridization (FISH), DNA sequencing, fluorescence microscopy, and flow cytometry. One way to achieve multicolor analysis is to label biomolecules such as nucleotides, proteins or DNA primers with different fluorescent markers and distinct fluorescence properties. Fluorophores with narrow emission bandwidths are preferred for multicolor labeling, because they have only a small overlap with the other dyes and hence increase the number of dyes possible in a multicolor experiment. Importantly, the emission maxima have to be well separated from each other to allow sufficient resolution of the signal. A suitable multicolor triplet of fluorophores would include Cy3, TRITC, and a photoluminescent compound as described herein, among others.

The simultaneous use of FISH (fluorescence *in-situ* hybridization) probes in combination with different fluorophores is useful for the detection of chromosomal translocations, for gene mapping on chromosomes, and for tumor diagnosis, to name only a few. One way to achieve simultaneous detection of multiple sequences is to use combinatorial labeling. Up to seven different DNA targets can be simultaneously visualized by using a combination of haptenated DNA probes (e.g. biotin, digoxigenin or dinitrophenol) with three sets of distinguishable fluorophores showing emission in the green (fluorescein), red (Texas Red), and blue (7-amino-4-methyl-coumarin-3-acidic acid or Cascade Blue) (Ried et al., Proc. Natl. Acad. Aci. USA 89, 1388-1392 (1992)). Three labeled DNA probes can be visualized by the distinct spectra of the three fluorescent markers, while four others will appear as fluorophore mixtures, e.g. probe 4 (fluorescein and rhodamine); probe 5 (fluorescein and Cascade Blue); probe 6 (rhodamine and cascade Blue); and probe 7 (fluorescein, rhodamine and Cascade Blue).

The second way is to label each nucleic acid probe with a fluorophore with distinct spectral properties. Similar conjugates can be synthesized from this invention and used in a multicolor multisequence analysis approach.

The luminescent compounds of the invention can also be used for screening assays for a combinatorial library of compounds. The compounds can be screened for a number of characteristics, including their specificity and avidity for a particular recognition moiety.

Assays for screening a library of compounds are well known. A screening assay is used to determine compounds that bind to a target molecule, and thereby create a signal change which is generated by a labeled ligand bound to the target molecule. Such assays allow screening of compounds that act as agonists or antagonists of a receptor, or that disrupt a protein-protein interaction. It also can be used to detect hybridization pr binding of DNA and/or RNA.

Other ways to do screening assays are based on compounds that affect the enzyme activity. For such purposes, nonfluorescent enzyme substrates of the invention could be used to trace the interaction with the substrate.

It is also possible to test for compounds that interfere with proteins that inhibit enzyme activity using an electrophoretic assay. In such assays, the most active compounds prevent enzyme inhibition, resulting in more enzymatic catalysis.

Besides non-fluorescent enzymes substrates, fluorescent substrates can be used for such assays. The cleavage of the fluorescent substrate leads to a change in the spectral properties such as the excitation and emission maximum, which allows to distinguish between the free and the bound fluorophore.

### Analytes

The invention can be used to detect an analyte that interacts with a recognition moiety in a detectable manner. As such, the invention can be attached to a recognition moiety which is known to those of skill in the art. Such recognition moieties allow the detection of specific analytes. Examples are pH-, or potassium sensing molecules, e.g., synthesized by introduction of potassium chelators such as crown-ethers (aza crowns, thia crowns etc). Calcium-sensors based on BAPTA (1,2-**B**is(2-**a**mino**p**henoxy)ethan-N,N,N',N'-tetra-aceticacic) as the chelating species are frequently used to trace the intracellular ion concentrations. The combination of a compound of the invention and the calcium binding moiety BAPTA can lead to new long-wavelength absorbing and emitting Ca-sensors which could be used for determination of intra- and extracellular calcium concentrations (Akkaya et al., Tetrahedron Lett. 38, 4513-4516 (1997)).

### Fluorescence methods

These compounds of the new invention can be detected applying commonly used intensity based fluorescent methods. The squaraine dyes are known to have lifetimes in the range of hundreds of ps to a few ns (see Table). The nanosecond lifetime and long-wavelength absorption and emission of these dyes when bound to proteins would allow them to be measured with inexpensive instrumentation using laser diodes for excitation and avalanche photodiodes for detection. Typical assay based on the measurement of the fluorescence lifetime as a parameter are FRET (fluorescence resonance energy transfer) assays. The binding between a fluorescent donor labeled species (typically an antigen) and a fluorescent acceptor labeled species are accompanied by a change in the intensity and the fluorescence lifetime. The lifetime can be measured using intensity- or phase-modulation-based methods (J.R. LAKOWICZ, PRINCIPLES OF FLUORESCENCE SPECTROSCOPY (1983)).

Squaraine dyes exhibit high intrinsic polarization in the absence of rotational motion, making them useful as tracers in fluorescence polarization (FP) assays. Fluorescence polarization immunoassays (FPI) are widely applied to quantify low molecular weight antigens. The assays are based on polarization measurements of antigens labeled with fluorescent probes. The requirement for polarization probes used in FPls is that emission from the unbound labeled antigen be depolarized and increase upon binding to the antibody. Low molecular weight species labeled with the compounds of the invention can be used in such binding assays, and the unknown analyte concentration can determined by the change in polarized emission from the fluorescent tracer molecule.

The materials, methods and applications of the invention are exemplified by the following description of the synthesis and the spectral properties of the compounds.

The synthesis and spectral characterization of a squaraine derivative for covalent attachment to biomolecules was already reported. Squaraines, which are 1,3-disubstituted squaric acid derivatives, show high absorption coefficients (ε > 200.000 l mol⁻¹ cm⁻¹) in the red region, display a high photostability, and allow the introduction of reactive functional groups such as NHS esters. These dyes exhibit good quantum yields on binding to proteins. The hydrophilic character of the dyes can be improved by introducing sulfonic acid groups.

The advantages of fluorescence detection at long wavelength excitation are the decreased autofluorescence from cells and tissues and the use of inexpensive laser light sources such as diode lasers operating at 635, 645, and 650 nm. The autofluorescence of biological samples decreases with increasing wavelength, particularly beyond 600 nm. Only a few fluorescent probes exist that absorb in the red or near infrared (NIR) region and even fewer of them are available in a reactive form. Amine-reactive functionalities such as isothiocyanate and N-hydroxysuccinimide esters and thiol-reactive iodoacetamide and maleimide groups can be used to covalently attach marker molecules to drugs, DNA, antibodies or synthetic polymers.

Although the invention has been disclosed in preferred forms, the specific embodiments thereof as disclosed and illustrated herein are not to be considered in a limiting sense, because numerous variations are possible.

## Claims

1. A composition of matter comprising a photoluminescent compound, the photoluminescent compound having the structure: 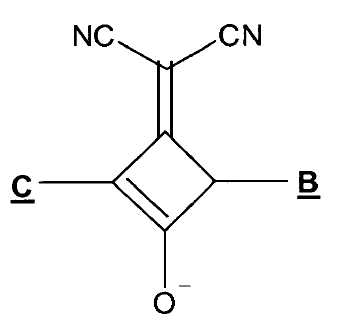 wherein:
(a)
each of B and C is selected from the group consisting of W¹ and W², wherein W¹ and W² are given by 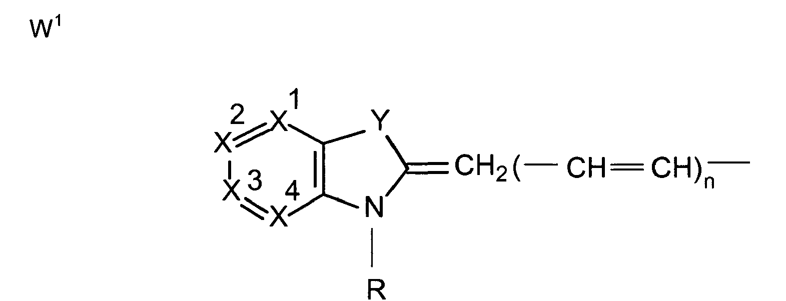 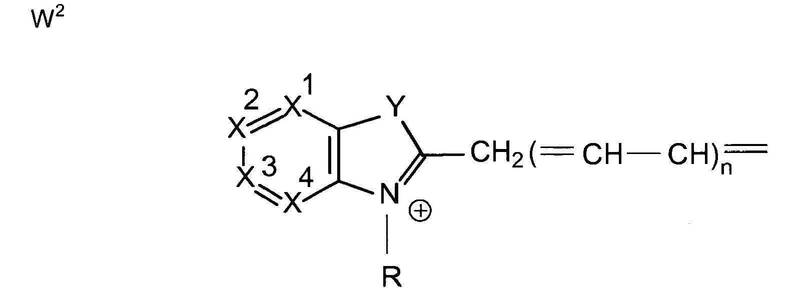
(b) one of B and C is W¹ and the other of B and C is W²;
(c) n is independently selected for each of B and C from the group consisting of 0, 1, and 2;
(d) Y is independently selected for each of B and C from the group consisting of O, S, Se, Te, N-R^{h}, and C(Rⁱ)(R^{j}), wherein R^{h} is selected from the group consisting of H, aliphatic groups, alicyclic groups and aromatic groups and wherein each of Rⁱ and R^{j} is selected from the group aliphatic groups;
(e) R is independently selected for each of B and C from the group consisting of H, aliphatic groups, alicyclic groups and aromatic groups; and
(f) each of X¹, X², X³, and X⁴ is independently selected for each of B and C from the group consisting of N, O, S, and C-R^{k}, wherein R^{k} is selected from the group consisting of H, F, Cl, Br, l, aliphatic groups, alicyclic groups, aromatic groups and parts of a substituted condensed aromatic or heterocyclic ring;
wherein the photoluminescent compound is covalently or non-covalently associated with a carrier that is a bead.

2. The composition of claim 1 wherein the photoluminescent compound is symmetric.

3. The composition of claim 1, wherein the photoluminescent compound is asymmetric.

4. The composition of claim 1 wherein n is 1.

5. A method of performing a photoluminescence assay, the method comprising:
exciting a photoluminescent compound as defined in any of claims 1 to 4 with excitation light; and
detecting emission light emitted by the photoluminescent compound.

## Patentansprüche

1. Stoffzusammensetzung, welche eine photolumineszente Verbindung umfasst, wobei die photolumineszente Verbindung die folgende Struktur aufweist: 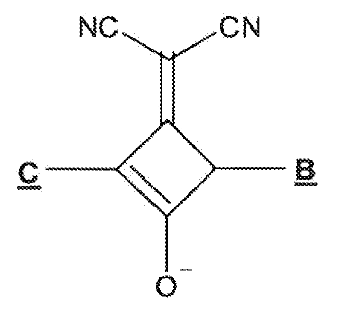 wobei:
(a)
B und C jeweils aus der aus W¹ und W² bestehenden Gruppe ausgewählt werden, wobei W¹ und W² gegeben sind durch 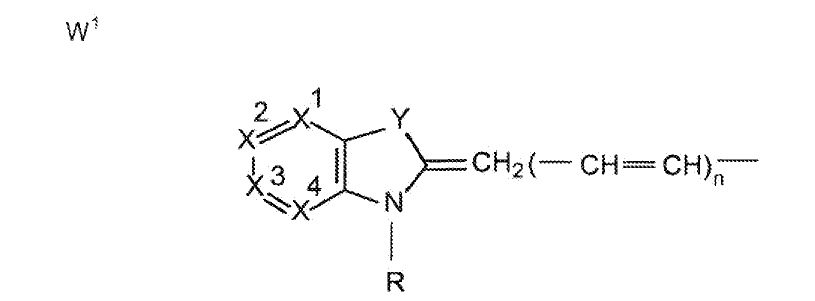 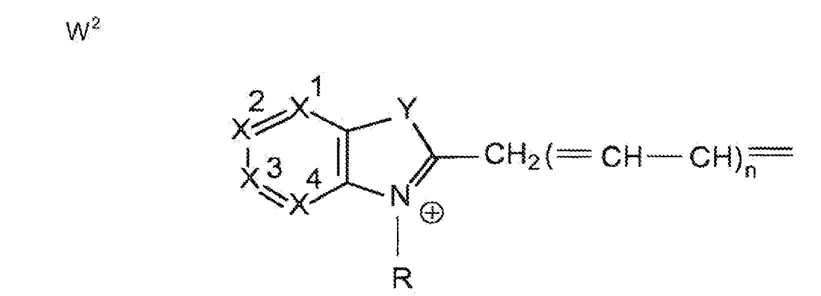
(b) B oder C W¹ ist und der jeweils andere Bestandteil W² ist;
(c) n für Bunt C jeweils unabhängig aus der Gruppe ausgewählt wird, die aus 0, 1 und 2 besteht;
(d) Y für B und C jeweils unabhängig aus der Gruppe ausgewählt wird, die aus O S, Se, Te, N-R^{h} und C(Rⁱ)(R^{j}) besteht, wobei R^{h} aus der Gruppe ausgewählt wird, die aus H, aliphatischen Gruppen, alizyklischen Gruppen und aromatischen Gruppen besteht, und wobei Rⁱ und R^{J} jeweils aus der Gruppe der aliphatischen Gruppen ausgewäh!t wird;
(e) R für B und C jeweils unabhängig aus der Gruppe ausgewählt wird, die aus H, aliphatischen Gruppen, alizyklischen Gruppen und aromatischen Gruppen besteht; und
(f) jeweils X¹, X², X³ und X⁴ für B und C jeweils unabhängig aus der Gruppe ausgewählt wird, die aus N, O, S und C-R^{k} besteht, wobei R^{k} aus der Gruppe ausgewählt wird, die aus H, F, Cl, Br, l, aliphatischen Gruppen, alizyklischen Gruppen, aromatischen Gruppen und Teilen eines substituierten kondensierten aromatischen oder heterocyklischen Rings besteht:
wobei die photolumineszente Verbindung kovalent oder non-kovalent an einen Träger gebunden ist, welcher eine Perle ist

2. Zusammensetzung nach Anspruch 1, wobei die photolumineszente Verbindung symmetrisch ist.

3. Zusammensetzung nach Anspruch 1, wobei die photolumineszente Verbindung asymmetrisch ist.

4. Zusammensetzung nach Anspruch 1, wobei n gleich 1 ist.

5. Verfahren zur Durchführung einer Photolumineszenz-Bestimmung, wobei das Verfahren Folgendes umfasst:
Anregen einer photolumineszenten Verbindung, wie sie in einem beliebigen der Ansprüche 1 bis 4 definiert ist, mit Anregungslicht; und
Erfassen von durch die photolumineszente Verbindung emittiertem Licht.

## Revendications

1. Composition de matière comprenant un composé photoluminescent, le composé photoluminescent ayant la structure : 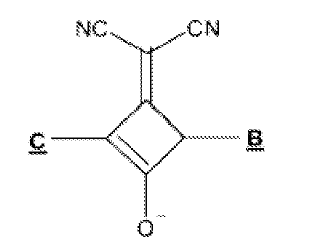 dans laquelle :
(a) chacun de B et C est choisi dans le groupe constitué par W¹ et W², W¹ et W² étant donnés par 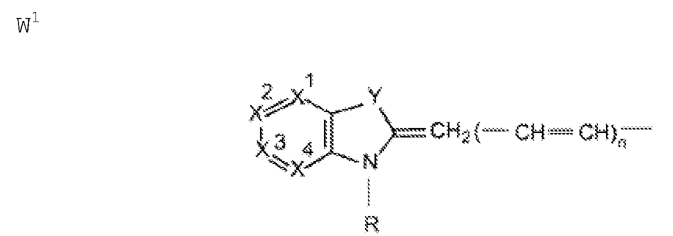 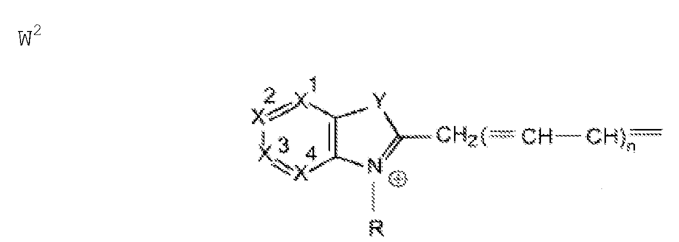
(b) l' un de B et C est W¹ et l'autre de B et C est W² ;
(c) n est choisi indépendamment pour chacun de B et C dans le groupe constitué par 0, 1 et 2 ;
(d) Y est choisi indépendamment pour chacun de B et C dans le groupe constitué par 0, S, Se, Te, N-R^{h} et C (R¹) (R^{j}) , R^{h} étant choisi dans le group constitué par H, les groupes aliphatiques, les groupes alicycliques et les groupes aromatiques et chacun de Rⁱ et R^{j} étant choisi dans le groupe des groupes aliphatiques ;
(e) R est choisi indépendamment pour chacun de B et C dans le groupe constitué par H, les groupes aliphatiques, les groupes alicycliques et les groupes aromatiques ; et
(f) chacun de X¹, X², X³ et X⁴ est choisi indépendamment pour chacun de B et C dans le groupe constitué par N, 0, S et C-R^{k} R^{k} étant choisi dans le groupe constitué par H, F, Cl, Br, I, les groupes aliphatiques, les groupes alicycliques, les groupes aromatiques et les parties d'un noyau aromatique ou hétérocyclique condensé substitué ;
le composé photoluminescent étant associé de manière covalente ou non-covalente à un support qui est une perle.

2. Composition selon la revendication 1, dans laquelle le composé photoluminescent est symétrique.

3. Composition selon la revendication 1, dans laquelle le composé photoluminescent est asymétrique.

4. Composition selon la revendication 1, dans laquelle n est 1.

5. Procédé de réalisation d'un essai de photoluminescence, le procédé comprenant :
- l'excitation d'un composé photoluminescent tel que défini à l'une quelconque des revendications 1 à 4 par une lumière d'excitation ; et
- la détection de la lumière d'émission émise par le composé photoluminescent.
